# EUROPEAN PATENT APPLICATION

(11) **EP 2 211 182 A1**
(43) Date of publication of application: **28.07.2010**
(21) Application number: 09150705.3
(22) Date of filing: 16.01.2009
(51) Int. Cl.: G01N 33/68, G01N 33/74

(54) **Method for the assessment of severity of liver cirrhosis**

(71) Applicant: Roche Diagnostics GmbH, 68305 Mannheim (DE); F. Hoffmann-La Roche AG, 4070 Basel (CH)
(72) Inventor: Hess, Georg, 55130 Mainz (DE); Horsch, Andrea, 68259 Mannheim (DE); Zdunek, Dietmar, 82327 Tutzing (DE)
(74) Representative: Dick, Alexander

(57) **Abstract**

The present invention is concerned with a method for diagnosing whether a subject suffers from a mild or severe form of liver cirrhosis based on determining the amount of GDF-15 and/or P1GF (Placental Growth Factor) in a sample from said subject and comparing the, thus, determined amount(s) with a reference amount (reference amounts). The method may further comprise determining the amount of adiponectin in a sample from said subject, and comparing said amount to a reference amount for adiponectin. Moreover, the present invention relates to identifying a subject being susceptible to liver transplantation comprising the amount of GDF-15 and/or PlGF (Placental Growth Factor)) in a sample from said subject and comparing the, thus, determined amount(s) with a reference amount (reference amounts). Further encompassed by the present invention are a kit and a device adapted to carry out the method of the present invention.

## Description

The present invention is concerned with a method for diagnosing whether a subject suffers from a mild or severe form of liver cirrhosis based on determining the amount of GDF-15 and/or PlGF (Placental Growth Factor) in a sample from said subject and comparing the, thus, determined amount(s) with a reference amount (reference amounts). The method may further comprise determining the amount of adiponectin in a sample from said subject, and comparing said amount to a reference amount for adiponectin. Moreover, the present invention relates to identifying a subject being susceptible to liver transplantation comprising the amount of GDF-15 and/or PlGF (Placental Growth Factor)) in a sample from said subject and comparing the, thus, determined amount(s) with a reference amount (reference amounts). Further encompassed by the present invention are a kit and a device adapted to carry out the method of the present invention.

The liver is the largest internal organ in the body and is involved in glycogen storage, degradation of red blood cells, plasma protein synthesis, and removal of drugs, alcohol and other harmful substances from the blood. Life without the liver is not possible; a subject can only survive a few hours without liver function.

The most abundant liver cells are hepatocytes which comprise approximately 70 % of the liver's mass. Blood supply for the liver comes from the hepatic artery, which supplies oxygen-rich blood. The liver also gets blood from the portal vein which filters blood from different organs.

Liver cirrhosis might evolve from liver fibrosis. It is a progressive, fibrosing and nodular condition disrupting the normal architecture of the liver. In liver cirrhosis liver tissue is replaced with non-living scar tissue as well as with regenerative nodules resulting in decreased liver function. Cirrhosis can be caused by the same disorders and drugs as liver fibrosis. Alcohol abuse is the most common cause for liver cirrhosis. Approximately 60 to 70 percent of newly diagnosed cases of cirrhosis are thought to be a consequence of chronic alcoholism (alcohol liver disease). Regardless of the cause of cirrhosis, the pathogenic features consist of the development of fibrosis to the point that there is architectural distortion with the formation of regenerative nodules surrounded by dense fibrotic tissue (see Harrison's Principles of Internal Medicine, 17th edition, editor A. Fauci, Mcgraw-Hill Professional).

Liver cirrhosis can be diagnosed by various diagnostic methods. The gold standard is liver biopsy for which a small sample from liver tissue is removed from the patient and analyzed under the microscope. Cirrhosis can also be diagnosed by various imaging techniques such as ultrasound, computerized tomography (CT) scan and magnetic resonance imaging (MRI).

Moreover, various blood tests for diagnosing liver cirrhosis were introduced during the last two decades (for a review, see Heidelbauch and Bruderly, 2006, American Family Physican, 74(5):756-762). These blood tests are based on the determination of the amount of certain polypeptides which are present or absent in liver cirrhosis. For example, cirrhosis results in reduced levels of blood albumin and in reduced levels of blood clotting factors as a consequence of the loss of the liver's ability to generate these proteins. Moreover, increased levels of the serum enzymes aspartate transaminase, alanin transaminase, alkaline phosphatase and gamma glutamyl-transferase may indicate liver cirrhosis. Another diagnostic assay it the bilirubin test. Bilirubin is a degradation product of the breakdown of blood cells. Impaired liver tissue can not sufficiently process bilirubin, leading to high blood levels of this pigment.

In order to make reliable decisions regarding the treatment of patients suffering from liver cirrhosis, the severity, i.e. the degree of liver cirrhosis needs to be carefully assessed (Staging/grading). Methods for grading liver cirrhosis are known in the art: By determining the Child-Pugh-Score liver cirrhosis can be classified into Child class A (relatively mild), Child class B, and Child class C (severe). The Child-Pugh-Score includes the determination of five empirically selected variables: two continuous variables (bilirubin and albumin) and three discrete variables (ascites, encephalopathy, and prothrombin time). Assessing the Child-Score, however, requires the determination of various markers/variables and, therefore, is expensive and time-consuming.

Staging liver cirrhosis by histologic examination of liver biopsies may result in complications of pain, bleeding, and even death (in rare cases). Liver biopsy is expensive, as are the costs associated with treating its complications. In addition, inter- and intraobserver error may lead to incorrect staging, as may sampling error in up to 33% of biopsies (see Adams et al., Clinical Chemistry. 2005;51:1867-1873.)

If the damage to the liver function is too severe and becomes life-threatening, liver transplantation is necessary. Although survival rate from liver transplantation has been improving within the last two decade, liver transplantation still puts the recipient at risk since approximately 20% of recipients die as a consequence of liver transplantation. Therefore, only patients with severe damage to liver function shall receive a liver transplant. For those patients, the benefits of transplantation (restoring liver function) will outweigh the disadvantages (risk of dying as a consequence of transplantation, high costs of liver transplantation).

The MELD system (model of end-stage liver disease) grades liver cirrhosis and allows for identifying those patients which most urgently need a liver transplant. MELD uses a formula based on three blood tests (bilirubin, creatinine and clotting (prothrombin time)) to generate a score for each patient. The score can range from 6 to 40. The higher the score, the greater the need of a patient for a transplant. The MELD system uses blood test results for bilirubin, creatinine and clotting (INR time). However, determining the MELD score is expensive and time consuming since the MELD score includes three variables.

Therefore, there is a clear long-standing need for means and methods allowing an assessment of the severity of liver cirrhosis. The said means and methods shall allow a reliable efficient diagnosis and shall avoid the drawbacks of the current techniques.

Thus, the technical problem underlying the present invention must be seen as the provision of means and methods for complying with the aforementioned needs.

The technical problem is solved by the embodiments characterized in the claims and herein below.

Accordingly, the present invention relates to a method for diagnosing whether a subject suffers from a mild or a severe form of liver cirrhosis,
a) determining the amount of GDF-15 and/or PlGF (Placental Growth Factor) in a sample from said subject,
b) comparing the amount(s) as determined in step a) with a reference amount (reference amounts), and
c) diagnosing whether said subject suffers from a mild or a severe form of liver cirrhosis based in the results obtained in step b).

The method of the present invention, preferably, is an in vitro method. Moreover, it may comprise steps in addition to those explicitly mentioned above. For example, further steps may relate to sample pre-treatments or evaluation of the results obtained by the method. The method of the present invention may be also used for monitoring, confirmation, and subclassification of patients suffering from liver cirrhosis. The method may be carried out manually or assisted by automation. Preferably, step (a) and/or (b) may in total or in part be assisted by automation, e.g., by a suitable robotic and sensory equipment for the determination in step (a) or a computer-implemented comparison in step (b).

The term "diagnosing" as used herein refers to assessing the severity of liver cirrhosis in a subject. Thus, the method allows grading cirrhosis. As will be understood by those skilled in the art, such an assessment is usually not intended to be correct for 100% of the subjects to be diagnosed. The term, however, requires that a statistically significant portion of subjects can be correctly diagnosed. Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the diagnosis will be correct for at least 60%, at least 70%, at least 80%, or at least 90% of the subjects of a given cohort or population.

The term "subject" as used herein relates to animals, preferably mammals, and, more preferably, humans. However, it is envisaged by the present invention that the subject shall be suffering from liver cirrhosis.

The term "liver cirrhosis" is understood by the skilled person. Liver cirrhosis is a liver disease in which the normal microcirculation, the gross vascular anatomy, and the hepatic architecture have been variably destroyed and altered with fibrous septa surrounding regenerated or regenerating parenchymal nodules. Liver cirrhosis might evolve from fibrosis. Thus, liver cirrhosis is a progressive, fibrosing and nodular condition disrupting the normal architecture of the liver. In liver cirrhosis liver tissue is replaced with non-living scar tissue as well as with regenerative nodules resulting in decreased liver function. Thus, cirrhosis occurs when the liver is inflamed and scar tissue and regenerative nodules form. Thus, liver cirrhosis occurs when pathogenic fibrosis develops to the point that there is architectural distortion in the liver with the formation of regenerative nodules surrounded by dense fibrotic tissue (see Harrison's Principles of Internal Medicine, 17th edition, editor A. Fauci, Mcgraw-Hill Professional, particularly chapter 302).

Liver cirrhosis can have various causes. Preferred causes for liver cirrhosis are chronic viral hepatitis B, chronic viral hepatitis C, alcoholic liver disease, non-alcoholic fatty liver disease, autoimmune liver disease (such as autoimmune hepatitis and primary biliary cirrhosis), inherited metabolic disorders (such as hematochromatosis, Wilson's disease, alfa 1-antitrypsin deficiency, and drug or toxin-induced liver disease.

The method of the present invention allows for diagnosing the severity of liver cirrhosis. Preferably, the method of the present invention allows for differentiating between a mild form and severe form of liver cirrhosis, i.e. for assessing whether a subject suffers from a mild or a severe form of liver cirrhosis. Preferably, a "mild form of liver cirrhosis" refers to a stage of liver cirrhosis graded as Child A by the Child-Pugh classification system. Preferably, a "severe form of liver cirrhosis" refers to a stage of liver cirrhosis graded as Child C by the Child-Pugh classification system.

The Child-Pugh classification system (also called Child-Pugh-Score or Child-Turcotte-Pugh) is well known in the art. Said system allows to allocate patients with liver cirrhosis into various groups depending on the severity of cirrhosis, see also Pugh et al., Transection of the oesophagus for bleeding oesophageal varices. Br J Surg 1973; 60:646-649). It is based on assessing five variables of liver cirrhosis, i.e. ascites, the plasma concentrations of bilirrubin and albumin, the prothrombin time, and the degree of encephalopathy.

It is to be understood that a subject which suffers from a mild form of liver cirrhosis has a larger life-expectancy than a subject which suffers from a severe form of liver cirrhosis.

Preferably, a subject with a mild form of liver cirrhosis has a remaining life-expectancy that ranges from 5 to 20 years, more preferably, from 10 to 20 years, and, most preferably, from 15 to 20 years. A subject with a severe form of liver cirrhosis, preferably, has a life-expectancy of 0 to 5 years, more preferably, of 1 to 4 years, and, most preferably, of 1 to 3 years.

The term "sample" refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein. More preferably, cell-, tissue- or organ samples are obtained from liver cells, liver tissue or the liver. The most preferred sample is the context of the present invention is a blood, blood serum, or a blood plasma sample.

The term "Growth-Differentiation Factor-15" or "GDF-15" relates to a polypeptide being a member of the transforming growth factor (TGF)-β cytokine superfamily. The terms polypeptide, peptide and protein are used interchangeable throughout this specification. GDF-15 was originally cloned as macrophage-inhibitory cytokine-1 and later also identified as placental transforming growth factor-β, placental bone morphogenetic protein, non-steroidal anti-inflammatory drug-activated genre-1, and prostate-derived factor (Bootcov loc cit; Hromas, 1997 Biochim Biophys Acta 1354:40-44; Lawton 1997, Gene 203:17-26; Yokoyama-Kobayashi 1997, J Biochem (Tokyo), 122:622-626; Paralkar 1998, J Biol Chem 273:13760-13767). Similar to other TGF-β-related cytokines, GDF-15 is synthesized as an inactive precursor protein, which undergoes disulfide-linked homodimerization. Upon proteolytic cleavage of the N-terminal pro-peptide, GDF-15 is secreted as an approx. 28 kDa dimeric protein (Bauskin 2000, Embo J 19:2212-2220). Amino acid sequences for GDF-15 are disclosed in WO99/06445, WO00/70051, WO2005/113585 Bottner 1999, Gene 237: 105-111, Bootcov loc. cit, Tan loc. cit., Baek 2001, Mol Pharmacol 59: 901-908, Hromas loc cit, Paralkar loc cit, Morrish 1996, Placenta 17:431-441 or Yokoyama-Kobayashi loc cit.. GDF-15 as used herein encompasses also variants of the aforementioned specific GDF-15 polypeptides. Such variants have at least the same essential biological and immunological properties as the specific GDF-15 polypeptides. In particular, they share the same essential biological and immunological properties if they are detectable by the same specific assays referred to in this specification, e.g., by ELISA assays using polyclonal or monoclonal antibodies specifically recognizing the said GDF-15 polypeptides. A preferred assay is described in the accompanying Examples. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific GDF-15 polypeptides. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Moreover, the variants referred to herein include fragments of the specific GDF-15 polypeptides or the aforementioned types of variants as long as these fragments have the essential immunological and biological properties as referred to above. Such fragments may be, e.g., degradation products of the GDF-15 polypeptides. Further included are variants which differ due to posttranslational modifications such as phosphorylation or myristylation.

The term "PIGF (Placental Growth Factor)" as used herein refers to a placenta derived growth factor which is a 149-amino-acid-long polypeptide and is highly homologous (53% identity) to the platelet-derived growth factor-like region of human vascular endothelial growth factor (VEGF). Like VEGF, PlGF has angiogenic activity in vitro and in vivo. For example, biochemical and functional characterization of PlGF derived from transfected COS-1 cells revealed that it is a glycosylated dimeric secreted protein able to stimulate endothelial cell growth in vitro (Maqlione1993, Oncogene 8(4):925-31). Preferably, PlGF refers to human PlGF, ore preferably, to human PlGF having an amino acid sequence as shown in Genebank accession number P49763, GI: 17380553 (Genebank is available from the NCBI, USA under www.ncbi.nlm.nih.gov/entrez) or a variant thereof. Moreover, it is to be understood that a variant as referred to in accordance with the present invention shall have an amino acid sequence which differs due to at least one amino acid substitution, deletion and/or addition wherein the amino acid sequence of the variant is still, preferably, at least 50%, 60%, 70%, 80%, 85%, 90%, 92%, 95%, 97%, 98%, or 99% identical with the amino sequence of the specific PlGF. How to determine the degree of identity is described elsewhere herein. Variants may be allelic variants, splice variants or any other species specific homologs, paralogs, or orthologs. Moreover, the variants referred to herein include fragments of the specific PlGF or the aforementioned types of variants as long as these fragments have the essential immunological and biological properties as referred to above. Such fragments may be, e.g., degradation products of PlGF. Further included are variants which differ due to posttranslational modifications such as phosphorylation or myristylation.

Determining the amount of the polypeptides referred to in this specification relates to measuring the amount or concentration, preferably semi-quantitatively or quantitatively. Measuring can be done directly or indirectly. Direct measuring relates to measuring the amount or concentration of the polypeptide based on a signal which is obtained from the polypeptide itself and the intensity of which directly correlates with the number of molecules of the polypeptide present in the sample. Such a signal - sometimes referred to herein as intensity signal -may be obtained, e.g., by measuring an intensity value of a specific physical or chemical property of the polypeptide. Indirect measuring includes measuring of a signal obtained from a secondary component (i.e. a component not being the polypeptide itself) or a biological read out system, e.g., measurable cellular responses, ligands, labels, or enzymatic reaction products.

In accordance with the present invention, determining the amount of a polypeptide can be achieved by all known means for determining the amount of a polypeptide in a sample.

Said means comprise immunoassay devices and methods which may utilize labeled molecules in various sandwich, competition, or other assay formats. Said assays will develop a signal which is indicative for the presence or absence of the polypeptide. Moreover, the signal strength can, preferably, be correlated directly or indirectly (e.g. reverse- proportional) to the amount of polypeptide present in a sample. Further suitable methods comprise measuring a physical or chemical property specific for the polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Preferably, determining the amount of a polypeptide comprises the steps of (a) contacting a cell capable of eliciting a cellular response the intensity of which is indicative of the amount of the polypeptide with the said polypeptide for an adequate period of time, (b) measuring the cellular response. For measuring cellular responses, the sample or processed sample is, preferably, added to a cell culture and an internal or external cellular response is measured. The cellular response may include the measurable expression of a reporter gene or the secretion of a substance, e.g. a peptide, polypeptide, or a small molecule. The expression or substance shall generate an intensity signal which correlates to the amount of the polypeptide.

Also preferably, determining the amount of a polypeptide comprises the step of measuring a specific intensity signal obtainable from the polypeptide in the sample. As described above, such a signal may be the signal intensity observed at an mass to charge (m/z) variable specific for the polypeptide observed in mass spectra or a NMR spectrum specific for the polypeptide.

Determining the amount of a polypeptide may, preferably, comprises the steps of (a) contacting the polypeptide with a specific ligand, (b) (optionally) removing non-bound ligand, (c) measuring the amount of bound ligand. The bound ligand will generate an intensity signal. Binding according to the present invention includes both covalent and non-covalent binding. A ligand according to the present invention can be any compound, e.g., a peptide, polypeptide, nucleic acid, or small molecule, binding to the polypeptide described herein. Preferred ligands include antibodies, nucleic acids, peptides or polypeptides such as receptors or binding partners for the polypeptide and fragments thereof comprising the binding domains for the peptides, and aptamers, e.g. nucleic acid or peptide aptamers. Methods to prepare such ligands are well-known in the art. For example, identification and production of suitable antibodies or aptamers is also offered by commercial suppliers. The person skilled in the art is familiar with methods to develop derivatives of such ligands with higher affinity or specificity. For example, random mutations can be introduced into the nucleic acids, peptides or polypeptides. These derivatives can then be tested for binding according to screening procedures known in the art, e.g. phage display. Antibodies as referred to herein include both polyclonal and monoclonal antibodies, as well as fragments thereof, such as Fv, Fab and F(ab)₂ fragments that are capable of binding antigen or hapten. The present invention also includes single chain antibodies and humanized hybrid antibodies wherein amino acid sequences of a non-human donor antibody exhibiting a desired antigen-specificity are combined with sequences of a human acceptor antibody. The donor sequences will usually include at least the antigen-binding amino acid residues of the donor but may comprise other structurally and/or functionally relevant amino acid residues of the donor antibody as well. Such hybrids can be prepared by several methods well known in the art. Preferably, the ligand or agent binds specifically to the polypeptide. Specific binding according to the present invention means that the ligand or agent should not bind substantially to ("cross-react" with) another peptide, polypeptide or substance present in the sample to be analyzed. Preferably, the specifically bound polypeptide should be bound with at least 3 times higher, more preferably at least 10 times higher and even more preferably at least 50 times higher affinity than any other relevant peptide or polypeptide. Non-specific binding may be tolerable, if it can still be distinguished and measured unequivocally, e.g. according to its size on a Western Blot, or by its relatively higher abundance in the sample. Binding of the ligand can be measured by any method known in the art. Preferably, said method is semiquantitative or quantitative. Suitable methods are described in the following.
First, binding of a ligand may be measured directly, e.g. by NMR or surface plasmon resonance.
Second, if the ligand also serves as a substrate of an enzymatic activity of the polypeptide of interest, an enzymatic reaction product may be measured (e.g. the amount of a protease can be measured by measuring the amount of cleaved substrate, e.g. on a Western Blot). Alternatively, the ligand may exhibit enzymatic properties itself and the "ligand/polypeptide" complex or the ligand which was bound by the polypeptide, respectively, may be contacted with a suitable substrate allowing detection by the generation of an intensity signal. For measurement of enzymatic reaction products, preferably the amount of substrate is saturating. The substrate may also be labeled with a detectable lable prior to the reaction. Preferably, the sample is contacted with the substrate for an adequate period of time. An adequate period of time refers to the time necessary for a detectable, preferably measurable, amount of product to be produced. Instead of measuring the amount of product, the time necessary for appearance of a given (e.g. detectable) amount of product can be measured.
Third, the ligand may be coupled covalently or non-covalently to a label allowing detection and measurement of the ligand. Labeling may be done by direct or indirect methods. Direct labeling involves coupling of the label directly (covalently or non-covalently) to the ligand. Indirect labeling involves binding (covalently or non-covalently) of a secondary ligand to the first ligand. The secondary ligand should specifically bind to the first ligand. Said secondary ligand may be coupled with a suitable label and/or be the target (receptor) of tertiary ligand binding to the secondary ligand. The use of secondary, tertiary or even higher order ligands is often used to increase the signal. Suitable secondary and higher order ligands may include antibodies, secondary antibodies, and the well-known streptavidin-biotin system (Vector Laboratories, Inc.). The ligand or substrate may also be "tagged" with one or more tags as known in the art. Such tags may then be targets for higher order ligands. Suitable tags include biotin, digoxygenin, His-Tag, Glutathion-S-Transferase, FLAG, GFP, myc-tag, influenza A virus haemagglutinin (HA), maltose binding protein, and the like. In the case of a peptide or polypeptide, the tag is preferably at the N-terminus and/or C-terminus. Suitable labels are any labels detectable by an appropriate detection method. Typical labels include gold particles, latex beads, acridan ester, luminol, ruthenium, enzymatically active labels, radioactive labels, magnetic labels ("e.g. magnetic beads", including paramagnetic and superparamagnetic labels), and fluorescent labels. Enzymatically active labels include e.g. horseradish peroxidase, alkaline phosphatase, beta-Galactosidase, Luciferase, and derivatives thereof. Suitable substrates for detection include di-amino-benzidine (DAB), 3,3'-5,5'-tetramethylbenzidine, NBT-BCIP (4-nitro blue tetrazolium chloride and 5-bromo-4-chloro-3-indolyl-phosphate, available as ready-made stock solution from Roche Diagnostics), CDP-Star™ (Amersham Biosciences), ECF™ (Amersham Biosciences). A suitable enzyme-substrate combination may result in a colored reaction product, fluorescence or chemoluminescence, which can be measured according to methods known in the art (e.g. using a light-sensitive film or a suitable camera system). As for measuring the enyzmatic reaction, the criteria given above apply analogously. Typical fluorescent labels include fluorescent proteins (such as GFP and its derivatives), Cy3, Cy5, Texas Red, Fluorescein, and the Alexa dyes (e.g. Alexa 568). Further fluorescent labels are available e.g. from Molecular Probes (Oregon). Also the use of quantum dots as fluorescent labels is contemplated. Typical radioactive labels include ³⁵S, ¹²⁵I, ³²P, ³³P and the like. A radioactive label can be detected by any method known and appropriate, e.g. a light-sensitive film or a phosphor imager. Suitable measurement methods according the present invention also include precipitation (particularly immunoprecipitation), electrochemiluminescence (electro-generated chemiluminescence), RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA), scintillation proximity assay (SPA), turbidimetry, nephelometry, latex-enhanced turbidimetry or nephelometry, or solid phase immune tests. Further methods known in the art (such as gel electrophoresis, 2D gel electrophoresis, SDS polyacrylamid gel electrophoresis (SDS-PAGE), Western Blotting, and mass spectrometry), can be used alone or in combination with labeling or other dectection methods as described above.

The amount of a polypeptide may be, also preferably, determined as follows: (a) contacting a solid support comprising a ligand for the polypeptide as specified above with a sample comprising the polypeptide and (b) measuring the amount of polypeptide which is bound to the support. The ligand, preferably chosen from the group consisting of nucleic acids, peptides, polypeptides, antibodies and aptamers, is preferably present on a solid support in immobilized form. Materials for manufacturing solid supports are well known in the art and include, inter alia, commercially available column materials, polystyrene beads, latex beads, magnetic beads, colloid metal particles, glass and/or silicon chips and surfaces, nitrocellulose strips, membranes, sheets, duracytes, wells and walls of reaction trays, plastic tubes etc. The ligand or agent may be bound to many different carriers. Examples of well-known carriers include glass, polystyrene, polyvinyl chloride, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention. Suitable methods for fixing/immobilizing said ligand are well known and include, but are not limited to ionic, hydrophobic, covalent interactions and the like. It is also contemplated to use "suspension arrays" as arrays according to the present invention (Nolan 2002, Trends Biotechnol. 20(1):9-12). In such suspension arrays, the carrier, e.g. a microbead or microsphere, is present in suspension. The array consists of different microbeads or microspheres, possibly labeled, carrying different ligands. Methods of producing such arrays, for example based on solid-phase chemistry and photo-labile protective groups, are generally known (US 5,744,305).

The term "amount" as used herein encompasses the absolute amount of a polypeptide, the relative amount or concentration of the said polypeptide as well as any value or parameter which correlates thereto or can be derived therefrom. Such values or parameters comprise intensity signal values from all specific physical or chemical properties obtained from the said polypeptides by direct measurements, e.g., intensity values in mass spectra or NMR spectra. Moreover, encompassed are all values or parameters which are obtained by indirect measurements specified elsewhere in this description, e.g., response levels determined from biological read out systems in response to the peptides or intensity signals obtained from specifically bound ligands. It is to be understood that values correlating to the aforementioned amounts or parameters can also be obtained by all standard mathematical operations.

Comparing as used herein encompasses comparing the amount of the polypeptides referred to herein which are comprised by the sample to be analyzed with an amount of the said polypeptides in a suitable reference sample as specified below in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison by means of an expert system. Accordingly, a diagnosis of the severity of liver cirrhosis referred to herein may be automatically provided in a suitable output format.

The term "reference amount" as used herein refers to an amount which allows for diagnosing whether a subject suffers from a mild form or severe form of liver cirrhosis as referred to above. Accordingly, the reference may either be derived from a subject known to suffer from a mild form of liver cirrhosis or from a subject known to suffer from a severe form of liver cirrhosis. It is to be understood that if a reference sample from a subject is used which suffers from a mild form of liver cirrhosis, an amount of the polypeptides in a sample of a test subject being essentially identical to the amounts determined in the said reference sample (i.e. the reference amounts) shall be indicative for a mild form of liver cirrhosis. Likewise, if a reference sample from a subject is used which suffers from a severe form of liver cirrhosis, an amount of the polypeptides in a sample of a test subject being essentially identical to the amounts determined in the said reference sample (i.e. the reference amounts) shall be indicative for a severe form of liver cirrhosis. The reference amount applicable for an individual subj ect may vary depending on various physiological parameters such as age, gender, or subpopulation. Thus, a suitable reference amount may be determined by the method of the present invention from a reference sample to be analyzed together, i.e. simultaneously or subsequently, with the test sample.

Moreover, a threshold amount can be preferably used as a reference amount. An amount of the polypeptides which is above the threshold amount or below the threshold amount, preferably will be indicative for either a mild or a severe form of liver cirrhosis. Preferably, an amount of the polypeptides which is above the threshold amount will be indicative for a severe form of liver cirrhosis. Preferably, an amount of the polypeptides which is below the threshold amount will be indicative for mild form of liver cirrhosis.

A preferred reference amount for GDF-15 in the context of the present invention defining a threshold amount for GDF-15 as referred to in accordance with the present invention is within a range of 4000 to 5000 pg/ml. More preferably, the reference amount is 4000 pg/ml, 4500 pg/ml or 5000 pg/ml.

Preferably, an amount of GDF-15 in a sample a subject larger than the reference amount for GDF-15 (defining a threshold amount for GDF-15) is indicative for a severe form of liver cirrhosis.

Preferably, an amount of GDF-15 in a sample a subject lower than the reference amount for GDF-15 (defining a threshold amount for GDF-15) is indicative for a mild form of liver cirrhosis.

A preferred reference amount for PlGF in the context of the present invention defining a threshold amount for PlGF as referred to in accordance with the present invention is within a range of 13 to 20 pg/ml, and more preferably, within of range of 15 to 18 pg/ml. Even more preferably, the reference amount is 15 pg/ml, 17 pg/ml or 20 pg/ml.

Preferably, an amount of PlGF in a sample a subject larger than the reference amount for PlGF (defining a threshold amount for PlGF) is indicative for a severe form of liver cirrhosis.

Preferably, an amount of PlGF in a sample a subject lower than the reference amount for PlGF (defining a threshold amount for PlGF) is indicative for a mild form of liver cirrhosis.

Advantageously, it was found in the studies underlying the present invention that determining at least one marker selected from the group consisting of GDF-15 and PlGF in a sample from a subject suffering from liver cirrhosis allows for efficiently and reliably diagnosing whether a subject suffers from a mild form or severe form of liver cirrhosis. Specifically, the amounts of GDF-15 and PlGF were determined in 31 subjects suffering from various forms of liver cirrhosis. It was shown that the amounts of the determined biomarkers were significantly increased in subjects suffering from liver cirrhosis graded Child class C compared with the amounts of said biomarkers in suffering from cirrhosis graded Child class A. Thus, determination of these biomarkers allows for reliably assessing the severity of liver cirrhosis in patients suffering from liver cirrhosis. Thanks to the method of the present invention, can be more readily and reliably diagnosed and subsequently correctly treated according to the result of the said differential diagnosis. The method of the present invention is particularly advantageous since it allows diagnosing whether a subject suffers from a mild form or severe form of liver cirrhosis by simply determining the amount of a marker in a sample from said subject. Particularly, an amount of the marker (GDF-15 and/or PlGF) in a sample from a subject suffering from liver cirrhosis larger than a suitable reference amount, preferably, indicates that said suffers from a severe form of liver cirrhosis, whereas an amount of the marker (GDF-15 and/or PlGF) in a sample from a subject suffering from liver cirrhosis larger than a suitable reference amount, preferably, indicates that said suffers from a mild form of liver cirrhosis.

In one embodiment of the method of the present invention only one marker is determined in a sample from the subject, i.e. GDF-15 or PlGF.

In another embodiment of the method of the present the amount of both markers, i.e. GDF-15 and PlGF, is determined. Determination of both markers enhances sensitivity and specificity of diagnosing whether a subject with liver cirrhosis suffers from a mild or severe form of liver cirrhosis.
Preferably, an amount of GDF-15 and PlGF in a sample from a subject suffering from liver cirrhosis lower than the corresponding reference amounts (and, thus, the reference amount of GDF-15 and PlGF, respectively) indicates that the subject suffers from a mild form of liver cirrhosis.

Preferably, an amount of GDF-15 and PlGF in a sample from a subject suffering from liver cirrhosis larger than the corresponding reference amounts (and, thus, the reference amount of GDF-15 and PlGF, respectively) indicates that the subject suffers from a severe form of liver cirrhosis.

Preferably, the method of the present invention further comprises determining the amount of adiponectin in said sample, and comparing said amount with a reference amount.

Adiponectin is a polypeptide (one of several known adipocytokines) secreted by the adipocyte. In the art, adiponectin is frequently also referred to as Acrp30 and apM1. Adiponectin has recently been shown to have various activities such as anti-inflammatory, antiatherogenic, preventive for metabolic syndrome, and insulin sensitizing activities. Adiponectin is encoded by a single gene, and has 244 amino acids, the molecular weight is approximately 30 kilodaltons. The mature human adiponectin protein encompasses amino acids 19 to 244 of full-length adiponectin. A globular domain is thought to encompass amino acids 107 - 244 of full-length adiponectin. The sequence of the adiponectin polypeptide is well known in the art, and, e.g., disclosed in WO/2008/084003.

Adiponectin associates itself into larger structures. Three adiponectin polypeptides bind together and form a homotrimer. These trimers bind together to form hexamers or dodecamers. Adiponectin is known to exist in a wide range of multimer complexes in plasma and combines via its collagen domain to create 3 major oligomeric forms: a low-molecular weight (LMW) trimer, a middle-molecular weight (MMW) hexamer, and high-molecular weight (HMW) 12- to 18-mer adiponectin (Kadowaki et al. (2006) Adiponectin and adiponectin receptors in insulin resistance, diabetes, and the metabolic syndrome. J Clin Invest. 116(7): 1784-1792; Rexford S. Ahima, Obesity 2006;14:242S-249S). Adiponectin has been reported to have several physiological actions, such as protective activities against atherosclerosis, improvement of insulin sensitivity, and prevention of hepatic fibrosis.

Adiponectin as used herein, preferably, relates to low molecular weight adiponectin, mid molecular weight adiponectin, more preferably, to total adiponectin, and, most preferably, to high molecular weight adiponectin. The terms high molecular weight adiponectin (12- to 18-mer adiponectin, preferably, 18-mer adiponectin), low and mid molecular weight adiponectin and total adiponectin are understood by the skilled person. Preferably, said adiponectin is human adiponectin. Methods for the determination of adiponectin are, e.g., disclosed in US 2007/0042424 A1 as well as in WO/2008/084003. Preferably, the amount of adiponectin is determined in a serum sample.

The adiponectin referred to in accordance with the present invention further encompasses allelic and other variants of said specific sequence for human adiponectin discussed above. Specifically, envisaged are variant polypeptides which are on the amino acid level preferably, at least 50%, 60%, 70%, 80%, 85%, 90%, 92%, 95%, 97%, 98%, or 99% identical, to human adiponectin. The degree of identity between two amino acid sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm of Smith and Waterman Add. APL. Math. 2:482 (1981), by the homology alignment algorithm of Needleman and Wunsch J. Mol. Biol. 48:443 (1970), by the search for similarity method of Pearson and Lipman Proc. Natl. Acad Sci. (USA) 85: 2444 (1988), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used. Variants referred to above may be allelic variants or any other species specific homologs, paralogs, or orthologs. Substantially similar and also envisaged are proteolytic degradation products which are still recognized by the diagnostic means or by ligands directed against the respective full-length peptide. Also encompassed are variant polypeptides having amino acid deletions, substitutions, and/or additions compared to the amino acid sequence of human adiponectin as long as the said polypeptides have adiponectin properties.

A preferred reference amount for adiponectin (preferably, of high molecular weight adiponectin) in the context of the present invention defining a threshold amount for adiponectin as referred to in accordance with the present invention is preferably, within a range of 5 to 9 µg/ml, and more preferably, within a range of 6 to 8 µg/ml. Even more preferably, the reference amount is 5.5 µg/ml, 6.5 µg/ml and, most preferably, 7.5 µg/ml.

Preferably, an amount of adiponectin (particularly of high molecular weight adiponectin) in a sample a subject larger than the reference amount for adiponectin (defining a threshold amount for adiponectin) is indicative for a severe form of liver cirrhosis provided that the amounts ofGDF-15 and/or PlGF are also larger than the reference amount.

Preferably, an amount of adiponectin in a sample a subject lower than the reference amount for adiponectin (defining a threshold amount for adiponectin) is indicative for a mild form of liver cirrhosis provided that the amounts of GDF-15 and/or PlGF are also lower than the reference amount.

Advantageously, it has been found in the studies carried out in the context of the present invention that the additional determination of adiponectin in a sample of the subject enhances the specificity and sensitivity of the diagnosis.

The explanations and definition given herein above apply mutatis mutandis to the following.

Moreover, the present invention also relates to a method for identifying a subject being eligible to liver transplantation, said subject suffering from liver cirrhosis, comprising the steps of
a) determining the amount of GDF-15 and/or PlGF (Placental Growth Factor) in a sample from said subject,
b) comparing the amount(s) as determined in step a) with a reference amount (reference amounts), and
c) identifying a subject being eligible to liver transplantation based on the results obtained in step b).

It has been found in the course of the present invention that the markers GDF-15 and PlGF (Placental Growth Factor) are reliable markers for diagnosing whether a subject suffers from a mild form or severe form of liver cirrhosis. Particularly, the said markers are increased in samples of subjects with severe liver cirrhosis compared with subjects with mild liver cirrhosis. Accordingly, the determination of said marker (alone or in combination) allows for identifying a subject being eligible to liver transplantation.

The phrase "identifying a subject being eligible to liver transplantation" as used herein refers to assessing whether a subject is susceptible/eligible to liver transplantation or not. A subject who is eligible to liver transplantation, preferably, will benefit from said transplantation.

Preferably, a subject benefits from liver transplantation if the advantages to said transplantation outweigh the disadvantages.

As described elsewhere herein, liver transplantation is a high risk, cost-intensive surgery, since some patients do not survive said surgery. Thus, disadvantages of liver transplantation are high costs and adverse side effects.

Liver transplantation, in the other hand, has the advantage that is allows for significantly increasing liver function and, therefore, may be required for survival. Accordingly, only patients suffering from severe forms of liver cirrhosis are eligible to liver transplantation, since the advantages of transplantation outweigh the disadvantages for those patients. If a patient suffers from a mild form of liver cirrhosis, said patient is, preferably, not susceptible to liver transplantation since the disadvantages of liver transplantation outweigh the advantages.

As will be understood by those skilled in the art, the described assessment is usually not intended to be correct for 100% of the subjects to be identified. The term, however, requires that a statistically significant portion of subjects can be correctly identified.

Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.1, 0.05, 0.01, 0.005, or 0.0001. Preferably, the probability envisaged by the present invention allows that the diagnosis will be correct for at least 60%, at least 70%, at least 80%, or at least 90% of the subjects of a given cohort or population.

The term "liver transplantation" is known by the person skilled in the art. Particularly, liver transplantation is a replacement of a diseased liver with a healthy liver allograft. Preferably, the term "liver transplantation" includes partial and whole liver transplantation". For liver transplantation, the liver of a donor is, preferably, resected (wholly or partially) and transplanted into a recipient. How to carry out a liver transplantation is well known in the art. Preferably, the donor is a dead donor. However, it is also contemplated that the donor is a living donor. Preferred methods for liver transplantation are, e.g., described by Reddy et al. (2004) Liver Transpl 10 (10): 1223-32, and Starzl et al. (1981) New England Journal of Medicine 305: 266-269.

Preferably, an amount of GDF-15 and/or PlGF (Placental Growth Factor) in sample from a subject suffering from liver cirrhosis larger than the (corresponding) reference amount is indicative for a subject being eligible to liver transplantation.

Preferably, an amount of GDF-15 and/or PlGF (Placental Growth Factor) in sample from a subject suffering from liver cirrhosis lower than the (corresponding) reference amount is indicative for a subject not being eligible to liver transplantation.

Preferred reference amounts are indicated herein above.

In a preferred embodiment the amount of only one marker as recited in the aforementioned method of the present invention is determined (and, thus, GDF-15 or PlGF). In another preferred embodiment the amount of GDF-15 and PlGF is determined.

Preferably, the aforementioned method further comprises determining the amount of adiponectin in said sample, and comparing said amount with a reference amount.

Preferably, an amount of adiponectin in a sample a subject larger than the reference amount for adiponectin (defining a threshold amount for adiponectin) is indicative for a subject being eligible to liver transplantation provided that the amounts of GDF-15 and/or PlGF are also larger than the reference amount.

Preferably, an amount of adiponectin in a sample a subject lower than the reference amount for adiponectin (defining a threshold amount for adiponectin) is indicative for a subject not being eligible to liver transplantation provided that the amounts of GDF-15 and/or PlGF are also lower than the reference amount.

It is to be undertstood that the determination of the amount of a biomarker (biomarkers) as recited herein also allows for monitoring a subject suffering from liver cirrhoris. For monitioring said subject, the amount of PlGF and/or GDF-15 is determined in a first sample of said subject and a second sample of said subject. In a further step, the amount of the marker(s) is said second sample is compared with the amount of said marker in said first sample. Preferably, an increase of the amount of PlGF and/or GDF-15 in said second sample compared with said first sample indicates that the cirrhosis has worsened. Preferably, a statistically significant increase indicates that the cirrhosis has significantly worsened (particularly, from a mild form of cirrhosis to a severe form of cirrhosis). Whether an increase is statically significant or not, can be determined by the skilled person without further ado.

Preferably, the second sample is obtained after the first sample. Preferred time intervals between obtaining said first and said second sample are 6 months, 1 year, 2 years, 3 years or five years.

The definitions and explanations given herein above apply mutatis mutandis to the following.

The present invention also relates to a method for diagnosing whether a subject suffers from i) a mild, ii) a moderate or iii) a severe form of liver cirrhosis, comprising the steps
a) determining the amount of GDF-15 and PlGF in a sample of said subject PlGF,
b) comparing the amounts of GDF-15 and PlGF as determined in step a) with a reference amount for GDF-15 and PlGF, and
c) diagnosing whether a subject suffers from i) a mild, ii) a moderate or iii) a severe form of liver cirrhosis,
   wherein
   i) an amount of GDF-15 and PlGF lower than the reference amounts for GDF-15 and PlGF indicate that said subject suffers from a severe from of liver cirrhosis,
   ii) an amount of GDF-15 larger than the reference amount for GDF-15, but an amount of PlGF lower than the reference amount for PlGF indicates that said subject suffers from a moderate form of liver cirrhosis, and
   iii) an amount of GDF-15 and PlGF larger than the reference amounts for GDF-15 and PlGF indicate that said subject suffers from a severe form of liver cirrhosis.

Definitions for the terms "mild form" and "severe form" of liver cirrhosis are given herein above. Preferably, the term "moderate form of liver cirrhosis" refers to a stage of liver cirrhosis graded as Child B by the Child-Pugh classification system.

Advantageously, it was shown that the determination of both PlGF and GDF-15 in sample from a subject with liver cirrhosis allows for differentiating between a mild, moderate and severe form of liver cirrhosis. Particularly, it was shown in the experiments carried out in the context of the present invention that amounts of PlGF and GDF-15 larger than respective reference amounts are indicative for a severe form of liver cirrhosis, whereas amounts of PlGF and GDF-15 lower than the respective reference amounts are indicative for a mild form of liver cirrhosis. Moreover, the amount of GDF-15 was increased in subjects with a moderate form of liver cirrhosis (Child B according to the Child-Pugh classification system), whereas the amount of PlGF was lower than the reference amount for PlGF (see figures).

Moreover, the present invention relates to a device and a kit adapted to carry out the method of the present invention.

Therefore, the present invention relates to a device for diagnosing whether a subject suffers from a mild form or severe form of liver cirrhosis, comprising means for determining the amount(s) of GDF-15 and/or PlGF (Placental Growth Factor) in a sample from said subject, and means for comparing said amount(s) with a reference amount (reference amounts).

Preferably, said device further comprises means for determining the amount of adiponectin and means for comparing said amount with a reference amount.

Furthermore, the present invention relates to a device for diagnosing whether a subject suffers from a mild form, moderate or severe form of liver cirrhosis, comprising means for determining the amounts of GDF-15 and PlGF (Placental Growth Factor) in a sample from said subject, and means for comparing said amounts with reference amounts.

Moreover, the present invention relates to a device for identifying a subject being eligible to liver transplantation, comprising means for determining the amount(s) of GDF-15 and/or PlGF (Placental Growth Factor) in a sample from said subject, and means for comparing said amount(s) with a reference amount (reference amounts).

Preferably, said device further comprises means for determining the amount of adiponectin and means for comparing said amount with a reference amount.

The term "device" as used herein relates to a system of means comprising at least the aforementioned means operatively linked to each other as to allow the prediction. Preferred means for determining the amount of the said polypeptides and means for carrying out the comparison are disclosed above in connection with the method of the invention. How to link the means in an operating manner will depend on the type of means included into the device. For example, where means for automatically determining the amount of the peptides are applied, the data obtained by said automatically operating means can be processed by, e.g., a computer program in order to diagnose or distinguish between the diseases referred to herein. Preferably, the means are comprised by a single device in such a case. Said device may accordingly include an analyzing unit for the measurement of the amount of the peptides in a sample and a computer unit for processing the resulting data for the differential diagnosis. Alternatively, where means such as test stripes are used for determining the amount of the polypeptides, the means for diagnosing may comprise control stripes or tables allocating the determined amount to an amount known to be accompanied with pulmonary embolism or pulmonary hypertension as the cause of right heart failure. The test stripes are, preferably, coupled to a ligand which specifically binds to the polypeptides as defined elsewhere in this specification. The strip or device, preferably, comprises means for detection of the binding of said peptides to the said ligand.

Preferred means for detection are disclosed in connection with embodiments relating to the method of the invention above. In such a case, the means are operatively linked in that the user of the system brings together the result of the determination of the amount and the diagnostic value thereof due to the instructions and interpretations given in a manual. The means may appear as separate devices in such an embodiment and are, preferably, packaged together as a kit. The person skilled in the art will realize how to link the means without further ado. Preferred devices are those which can be applied without the particular knowledge of a specialized clinician, e.g., test stripes or electronic devices which merely require loading with a sample. The results may be given as output of parametric diagnostic raw data, preferably, as absolute or relative amounts. It is to be understood that these data will need interpretation by the clinician. However, also envisaged are expert system devices wherein the output comprises processed diagnostic raw data the interpretation of which does not require a specialized clinician. Further preferred devices comprise the analyzing units/devices (e.g., biosensors, arrays, solid supports coupled to ligands specifically recognizing the polypeptides, Plasmon surface resonace devices, NMR spectrometers, mass- spectrometers etc.) or evaluation units/devices referred to above in accordance with the method of the invention.

Furthermore, the present invention relates to a kit for diagnosing whether a subject suffers from a mild form, or severe form of liver cirrhosis, said kit comprising instructions for carrying out the diagnosis, and means for determining the amount(s) of GDF-15 and/or PlGF (Placental Growth Factor) in a sample from said subject, and means for comparing said amount(s) with a reference amount (reference amounts).

Preferably, said kit further comprises means for determining the amount of adiponectin and means for comparing said amount with a reference amount.

Furthermore, the present invention relates to a kit for identifying a subject being eligible to liver transplantation, said kit comprising instructions for carrying out the identification, and means for determining the amount(s) of GDF-15 and/or PlGF (Placental Growth Factor) in a sample from said subject, and means for comparing said amount(s) with a reference amount (reference amounts).

Preferably, said kit further comprises means for determining the amount of adiponectin and means for comparing said amount with a reference amount.

Furthermore, the present invention relates to a kit for diagnosing whether a subject suffers from a mild form, moderate or severe form of liver cirrhosis, said kit comprising instructions for carrying out the diagnosis, and means for determining the amounts of GDF-15 and PlGF (Placental Growth Factor) in a sample from said subject, and means for comparing said amounts with reference amounts.

The term "kit" as used herein refers to a collection of the aforementioned means, preferably, provided in separately or within a single container. The container, also preferably, comprises instructions for carrying out the method of the present invention. The invention, thus, relates to a kit comprising a means or an agent for measuring a polypeptide referred to herein. Examples for such means or agents as well as methods for their use have been given in this specification. The kit, preferably, contains the aforementioned means or agents in a ready-to-use manner. Preferably, the kit may additionally comprise instructions, e.g., a user's manual for interpreting the results of any determination(s) with respect to the diagnoses provided by the methods of the present invention. Particularly, such manual may include information for allocating the amounts of the determined polypeptides to the kind of diagnosis. Details are to be found elsewhere in this specification. Additionally, such user's manual may provide instructions about correctly using the components of the kit for determining the amount(s) of the respective biomarker. A user's manual may be provided in paper or electronic form, e.g., stored on CD or CD ROM. The present invention also relates to the use of said kit in any of the methods according to the present invention.

Also, present invention relates to the use of GDF-15 and/or PlGF in a sample from a subject with liver cirrhosis, for diagnosing whether said subject suffers from a mild form or a severe form of liver cirrhosis, or for identifying a subject being eligible for liver transplantation.

Moreover, the present invention relates to the use of PlGF and adiponectin in a sample from a subject with liver cirrhosis, for diagnosing whether said subject suffers from a mild form or a severe form of liver cirrhosis, or for identifying a subject being eligible for liver transplantation.

Also, the present invention relates to the use of GDF-15 and adiponectin in a sample from a subject with liver cirrhosis, for diagnosing whether said subject suffers from a mild form or a severe form of liver cirrhosis, or for identifying a subject being eligible for liver transplantation.

Also, the present invention relates to the use of GDF-15, PlGF and adiponectin in a sample from a subject with liver cirrhosis, for diagnosing whether said subject suffers from a mild form or a severe form of liver cirrhosis, or for identifying a subject being eligible for liver transplantation.

Finally, the present invention relates to the use of GDF-15 and PlGF in a sample from a subject with liver cirrhosis, for diagnosing whether said subject suffers from a mild, moderate, or a severe form of liver cirrhosis,

All references cited in this specification are herewith incorporated by reference with respect to their entire disclosure content and the disclosure content specifically mentioned in this specification.

### The figures show:

**Fig 1**: GDF-15 levels in subjects with liver fibrosis or liver cirrhosis (Box plot diagram, see also Example 1)
**Fig 2**: PlGF levels in subjects with liver fibrosis or liver cirrhosis (Box plot diagram, see also Example 1)
**Fig 3**: PlGF levels in subjects with liver fibrosis or liver cirrhosis (Box plot diagram, see also Example 1)

The following Examples merely illustrate the invention. It shall, whatsoever, not be construed as to limit the scope of the invention.

### Example 1

The amounts of high molecular weight adiponectin, GDF-15 and PlGF were determined in a total of 64 patients suffering liver fibrosis and liver cirrhosis.

The patient collective is the same as decribed by Raedle-Hurst et al. (Europoean Journal of Gastroenterology & Hepatology 2008, Vol 20 No 9).

Mean age was 48.1 +/- 6.5 years (range 31-65). Twenty six patients were female and 38 were male.

A hepatic biopsy was performed in all patients except those with manifest signs of liver cirrhosis (see Raedle-Hurst et al., loc. cit). Liver biopsy specimens were assessed by an experienced pathologist who was unaware of the clinical and biochemical data of the patients. Fibrosis of the liver was staged according to the Ishak score (Ishak et al., Histological grading and staging of chronic hepatitis. J Hepatol 1995; 22:696-699). Mild fibrosis refers to stage F1-2, moderate fibrosis to F3-4, and severe fibrosis/cirrhosis to F5-6. Severe fibrosis/cirrhosis was classified according to the Child-Turcotte-Pugh criteria (Pugh et al., Transection of the oesophagus for bleeding oesophageal varices. Br J Surg 1973; 60:646-649).

Staging of liver disease indicated mild fibrosis (F1-2) in 25 of 64 (39.1%) patients, moderate fibrosis (F3-4) in 8 of 64 (12.5%) patients, and severe fibrosis/cirrhosis (F5-6) in 31 of 64 (48.4%) patients. In the subgroup of patients with severe fibrosis/cirrhosis, the degree of liver dysfunction was classified as Child-Turcotte-Pugh class A in 11 patients, B in 11 patients, and C in 9 patients.

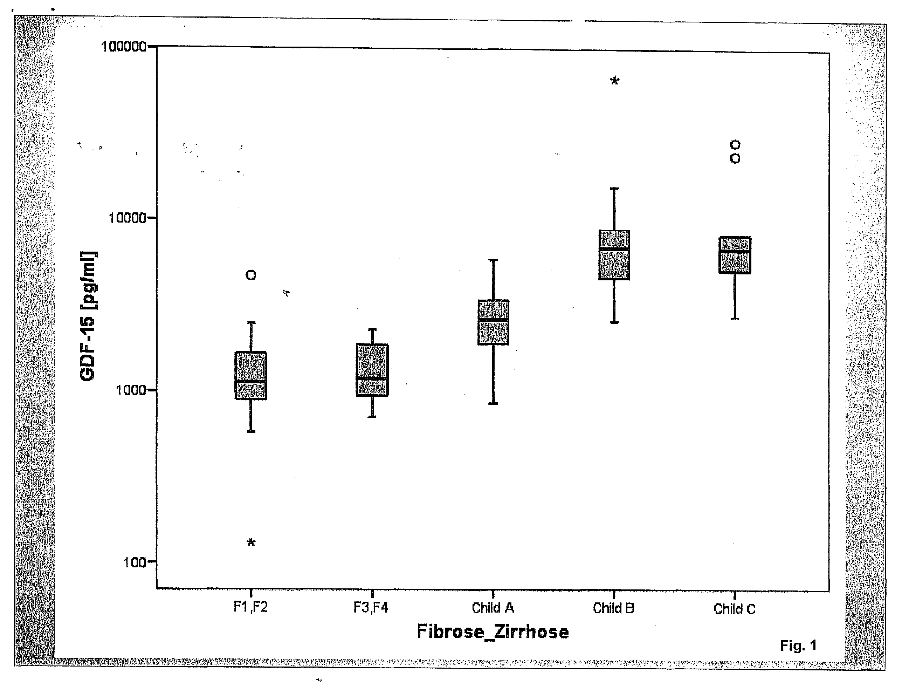

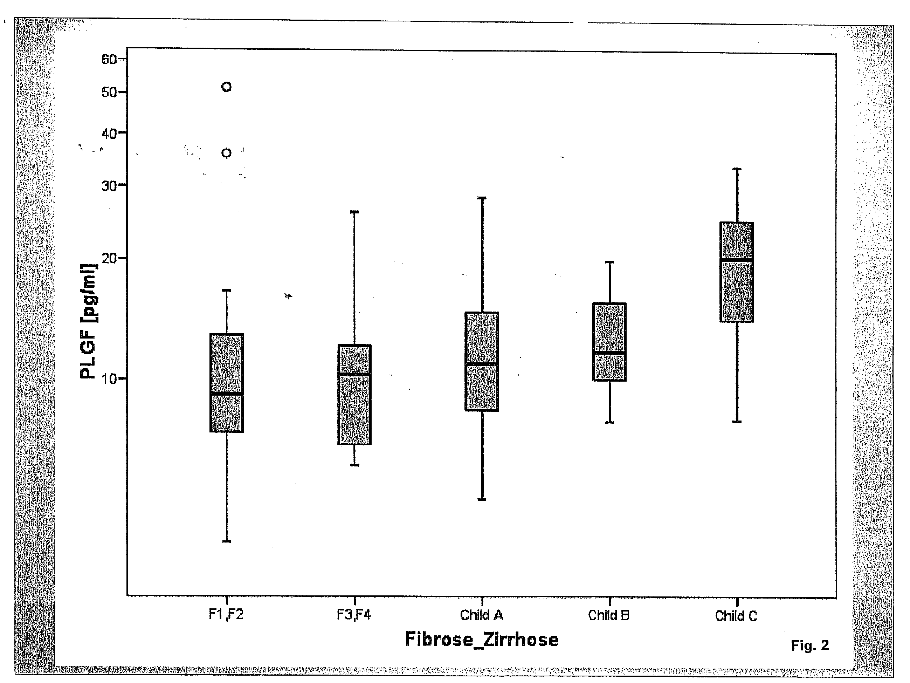

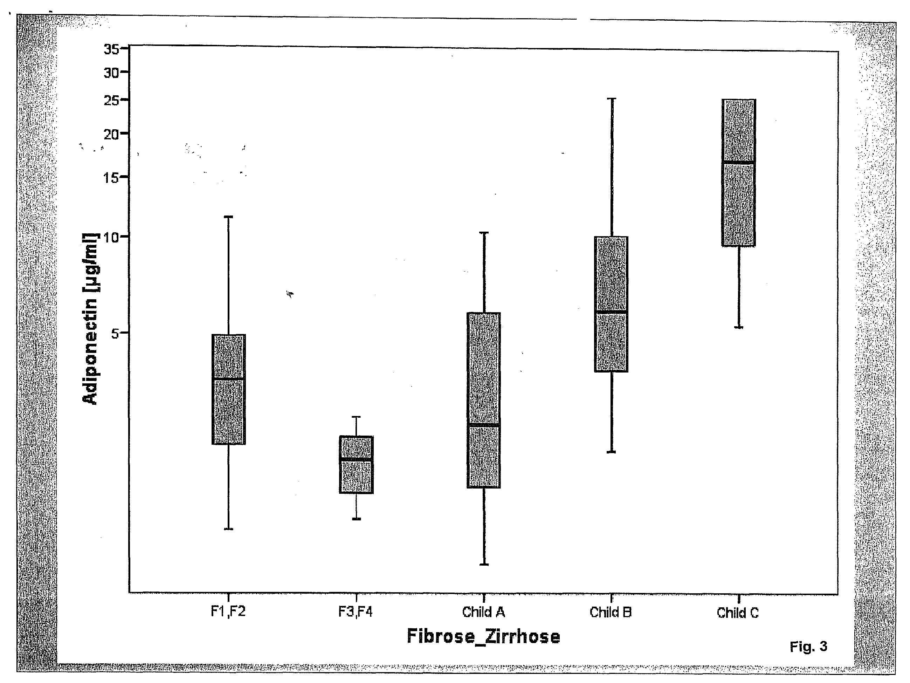

The results of the determination of the various markers are shown are shown in Fig. 1 to Fig. 3.

As it can be seen from the results, the amounts of adiponectin, GDF-15 and PlGF are larger in samples from patients with severe cirrhosis than in samples from patients with mild cirrhosis. Particularly, the amount of GDF-15 was increased in patients with cirrhosis graded Child B and C compared with the amount of GDF-15 in patients with cirrhosis graded Child A. Moreover, the amount of PlGF was increased in patients with cirrhosis graded Child C compared with the amount of PlGF in patients with cirrhosis graded Child A and B. Accordingly, the determination of these markers allows for differentiating between the aforementioned grades of cirrhosis.

### Example 2

A 62 years old patient presents at his primary care physician. PlGF (12.1 pg/ml) and GDF-15 (2780 pg/ml) are determined in a serum sample from said subject. A liver biopsy is carried out and subsequently subjected to histologic examination. Small regenerative nodules are detected indicating liver cirrhosis. Cirrhosis is staged by determining the Child-Pugh-Score (Child A).

### Example 3

After an acute episode of liver cirrhosis in a subject with advanced liver disease, a decision has to be made whether said subject requires a liver transplant. Cirrhosis is staged by determining the Child-Pugh-Score (Child C). Moreover, PlGF (18 pg/ml) and GDF-15 (6850 pg/ml), and high molecular weight adiponectin (15.2 µg/ml) are determined in a serum sample from the patient indicating that the subject suffers from end-stage liver disease (severe form of liver cirrhosis). Therefore, the patient is enlisted for liver transplantation. Liver transplantation is carried out after six weeks.

### Example 4

GDF-15 was determined by an electrochemoluminescence immunoassay the Elecsys^{™} instrument (Roche Diagnostics GmbH, Mannheim, Germany). The assay works according to the electrochemoluminescence sandwich immunoassay principle. In a first step, the biotin-labelled IgG (1-21) capture antibody, the ruthenium-labelled F(ab')₂ (39-50) signal antibody and 20 microliters of sample are incubated at 37 °C for 9 minutes. Afterwards, streptavidin-coated magnetic microparticles are added and the mixture is incubated for additional 9 minutes. After the second incubation, the reaction mixture is transferred to the measuring cell of the system where the beats are magnetically captured onto the surface of an electrode. Unbound label is removed by washing the measuring cell with buffer.

In the last step, voltage is applied to the electrode in the presence of a tri-propylamine containing buffer and the resulting electrochemoluminescent signal is recorded by a photomultiplier. All reagents and samples are handled fully automatically by the Elecsys^{™} instrument. Results are determined via a calibration curve which is instrument-specifically generated by 2-point calibration and a master curve provided via the reagent barcode. The test is performed according to the instructions of the manufacturer.

Blood levels of PlGF were determined using the commercially available Elecsys Immunoassays from Roche Diagnostics, DE.

Blood levels of adiponectin were determined in serum samples by using the commercially available ALPCO adiponectin multimeric EIA immunoassay for adiponectin.

## Claims

1. A method for diagnosing whether a subject suffers from a mild or a severe form of liver cirrhosis,
a) determining the amount of GDF-15 and/or PlGF (Placental Growth Factor) in a sample from said subject,
b) comparing the amount(s) as determined in step a) with a reference amount (reference amounts), and
c) diagnosing whether said subject suffers from a mild or a severe form of liver cirrhosis based on the results of step b).

2. The method of claim 1, wherein said subject is a human.

3. The method of claims 1 and 2, wherein said sample is blood, blood serum or blood plasma.

4. The method of any one claims of 1 to 3, wherein the amount of one marker (GDF-15 or PlGF) is determined.

5. The method of claim of any one of claims 1 to 4, wherein an amount of said marker larger than the reference amount is indicative for a severe form of liver cirrhosis, and/or wherein an amount of said marker lower than the reference amount is indicative for a mild form of liver cirrhosis.

6. The method of claim 5, wherein the marker is GDF-15 and the reference amount for GDF-15 is 4500 pg/ml.

7. The method of claim 5, wherein the marker is PlGF and the reference amount for PlGF is 17 pg/ml.

8. The method of any one of claims of 1 to 3, wherein the amounts of GDF-15 and PlGF are determined.

9. The method of claim 5, further comprising determining the amount of adiponectin in said sample and comprising the, thus, determined amount with a reference amount for adiponectin.

10. A method for identifying a subject being eligible to liver transplantation, said subject suffering from liver cirrhosis, comprising the steps of
a) determining the amount of GDF-15 and/or PlGF (Placental Growth Factor) in a sample from said subject,
b) comparing the amount(s) as determined in step a) with a reference amount (reference amounts), and
c) identifying a subject being eligible to liver transplantation based on the results of step b).

11. The method of claim 10, wherein an amount of said at least one marker larger than the reference amount indicates that said subject is eligible to liver transplantation and/or wherein an amount of said at least one marker lower than the reference amount indicates that said subject is not eligible to liver transplantation.

12. A device for diagnosing whether a subject suffers from a mild form or severe form of liver cirrhosis, comprising means for determining the amount(s) of GDF-15 and/or PlGF (Placental Growth Factor) in a sample from said subject, and means for comparing said amount(s) with a reference amount (reference amounts).

13. A device for identifying a subject being eligible to liver transplantation, comprising means for determining the amount(s) of GDF-15 and/or PlGF (Placental Growth Factor) in a sample from said subject, and means for comparing said amount(s) with a reference amount (reference amounts).

14. A kit for whether a subject suffers from a mild form or severe form of liver cirrhosis, said kit comprising instructions for carrying out the diagnosis, and means for determining the amount(s) of GDF-15 and/or PlGF (Placental Growth Factor) in a sample from said subject, and means for comparing said amount(s) with a reference amount (reference amounts).

15. A kit for identifying a subject being eligible to liver transplantation, said kit comprising instructions for carrying out the diagnosis, and means for determining the amount(s) of GDF-15 and/or PlGF (Placental Growth Factor) in a sample from said subject, and means for comparing said amount(s) with a reference amount (reference amounts).

16. The kit of device of claim 12 or 13, or the kit of claim 14 or 15, further comprising means for determining the amount of adiponectin, and, means for comparing said amounts with a reference amount for adiponectin.
